# EUROPEAN PATENT SPECIFICATION

(11) **EP 3 512 460 B1**
(45) Date of publication and mention of the grant of the patent: **01.11.2023**
(21) Application number: 17851603.5
(22) Date of filing: 15.09.2017
(51) Int. Cl.: A61F 2/06, A61L 27/26, A61L 27/56, A61L 27/38, A61L 27/50, D01D 5/00, D01F 6/88, D06M 15/15

(54) **ENGINEERED BLOOD VESSELS**
GEZÜCHTETE BLUTGEFÄSSE
VAISSEAUX SANGUINS OBTENUS PAR INGÉNIERIE

(30) Priority: 16.09.2016 US 201662395703 P
(43) Date of publication of application: 24.07.2019
(73) Proprietor: University of Kansas, Lawrence KS 66045 (US)
(72) Inventor: QUINT, Clay, Prairie Village, Kansas 66208 (US)
(74) Representative: Deambrogi, Edgardo
(86) International application number: PCT/US2017/051777
(87) International publication number: WO 2018/053265

(56) References cited:
- US-A1- 2006 204 539
- US-A1- 2006 240 061
- US-A1- 2006 263 417
- US-A1- 2011 311 746
- US-A1- 2014 058 496
- US-A1- 2014 322 512
- US-A1- 2015 112 419
- US-B1- 6 197 296
- PETRA E DIJKMAN ET AL: "Decellularized homologous tissue-engineered heart valves as off-the-shelf alternatives to xeno- and homografts", BIOMATERIALS, ELSEVIER, AMSTERDAM, NL, vol. 33, no. 18, 4 March 2012 (2012-03-04) , pages 4545-4554, XP028411029, ISSN: 0142-9612, DOI: 10.1016/J.BIOMATERIALS.2012.03.015 [retrieved on 2012-03-12]
- QUINT ET AL.: 'Decellularized Tissue-Engineered Blood Vessel as an Arterial Conduit' PROCEEDINGS OF THE NATIONAL ACADEMY OF SCIENCES vol. 108, 31 May 2011, pages 9214 - 9219, XP055078285
- NAM ET AL.: 'Improved Cellular Infiltration in Electrospun Fiber via Engineered Porosity' TISSUE ENGINEERING vol. 13, 01 September 2007, pages 2249 - 2257, XP055207535

## Description

### RELATED APPLICATIONS

This application claims the benefit of priority to U.S. Provisional Application Number 62/395,703, filed September 16, 2016.

### STATEMENT REGARDING FEDERALLY SPONSORED RESEARCH OR DEVELOPMENT

This invention was made with Government Support under Grant No. TR000001 awarded by the National Institutes of Health. The Government has certain rights in the invention.

### BACKGROUND

Cardiovascular-related disorders are a leading cause of death in developed countries. In the United States alone, one cardiovascular death occurs every 34 seconds and cardiovascular disease-related costs are approximately $250 billion. Current methods for treatment of vascular disease include chemotherapeutic regimens, angioplasty, insertion of stents, reconstructive surgery, bypass grafts, resection of affected tissues, or amputation. Unfortunately, for many patients, such interventions show only limited success, and many patients experience a worsening of the conditions or symptoms.

The treatment of cardiovascular diseases often requires reconstruction and replacement of blood vessels. Currently, the most popular source of replacement vessels is autologous arteries and veins. However, such autologous vessels are in short supply or are not suitable especially in patients who have had vessel disease or previous surgeries.

Synthetic grafts made of materials such, as PTFE and Dacron are popular vascular substitutes. Despite their popularity, synthetic materials are not suitable for small diameter grafts or in areas of low blood flow. Material-related problems such as stenosis, thromboembolization, calcium deposition, and infection have also been demonstrated.

To overcome these deficiencies, there is a need for improved grafts that can be used to treat vascular diseases and disorders.

US 2015/112419 describes an engineered vascular graft comprising a tubular scaffold which is formed by electrospinning a matrix of nanofibers comprising a mixture of a natural polymer component and a synthetic polymer component. A porogen can be used to create sufficient porosity. A cell sheet of smooth muscle cells is applied around the construct and cultured under pulsed fluid flow. Endothelial cells can additionally be seeded on the inside of the graft.

### SUMMARY

The scope of this invention is defined by the claims. Provided herein are methods of forming grafts (e.g., autologous vascular grafts, biliary conduits, ureter conduits, etc.) for implantation into subjects in need thereof. The methods employ an electrospun scaffold formed from nanofibers that comprise a blend of a biodegradable synthetic polymer and a biopolymer. Extracellular matrix-producing cells (e.g., fibroblasts) are then cultured on the electrospun scaffold in a bioreactor to form a cellular, tubular structure. The tubular structure is then decellularized, and endothelial cell from the subject are cultured on the resulting decellularized scaffold to form a graft (e.g., autologous vascular grafts) for implantation into a subject. Because decellularized scaffolds can be prepared in advance, the methods described herein can be used to prepare autologous grafts for implantation in a subject in a relatively short, clinically relevant timeframe (e.g., from one week to one month).

Methods of forming grafts for implantation into a subject comprise (i) forming an electrospun scaffold comprising a substantially tubular matrix formed from nanofibers that comprise a blend of a biodegradable synthetic polymer and a biopolymer, wherein the tubular matrix comprises an external surface, an internal surface, and a lumen extending therethrough, wherein the nanofibers have a diameter of from 50 nm to 2000 nm, wherein the tubular matrix has a porosity of at least 50%, as determined by mercury porosimetry or apparent density; (ii) culturing a population of extracellular matrix-producing cells on the electrospun tubular scaffold to form a tissue-engineered tubular construct; (iii) decellularizing the tissue-engineered tubular construct to form a tissue-engineered scaffold; and (iv) culturing endothelial cells from the subject on the tissue-engineered scaffold to form a graft for implantation into the subject.

The nanofibers can comprise from 60-80% by weight biodegradable synthetic polymer and from 10-40% by weight biopolymer. The biodegradable synthetic polymer can comprise, for example, a polyester such as polylactic acid (PLA), polyglycolic acid (PGA), poly lactic-co-glycolide (PLGA), polycaprolactone (PCL), polydioxanone (PDS), a polyhydroxyalkanoate (PHA), polyurethane (PU), copolymers thereof, and blends thereof. Other suitable biodegradable synthetic polymers include, for example, polyurethanes. In certain embodiments, the biodegradable synthetic polymer can comprise PGA. The biopolymer can comprise, for example, a peptide or protein such as gelatin, collagen, elastin, silk fibroin, and combinations thereof. In certain embodiments, the biodegradable synthetic polymer can comprise gelatin. In some embodiments, the biodegradable synthetic polymer comprises polyglycolic acid (PGA) and the biopolymer comprises gelatin. The nanofibers can have a diameter of from 50 nm to 2000 nm. e.g., from 50 nm to 1000 nm, from 50 nm to 500 nm, from 500 nm to 2000 nm, or from 500 nm to 1000 nm). In some cases, the electrospun scaffold can be substantially free of crosslinkers such as glutaraldehyde.

The electrospun scaffold can be prepared by a method that comprises electrically charging a first solution comprising the biodegradable synthetic polymer and the biopolymer; electrically charging a second solution comprising a porogen; discharging the electrically charged first solution and the electrically charged second solution onto a grounded target (e.g., a rotating mandrel) under an electrostatic field, such that the movement of the electrically charged first solution under the electrostatic field causes the electrically charged first solution to evaporate and produce the nanofibers that form the tubular matrix on the grounded target, and the movement of the electrically charged second solution under the electrostatic field causes the electrically charged second solution to evaporate and produce particles comprising the porogen amongst the nanofibers on the grounded target; and removing the particles comprising the porogen from amongst the nanofibers to form the electrospun scaffold.

In some embodiments, the grounded target can comprise a rotating mandrel. The dimensions of the mandrel and the parameters of the electrospinning process can be varied based on the desired dimensions of the electrospun scaffold (and by extension the resulting graft).

In some embodiments, the electrically charged first solution and the electrically charged second solution can both be discharged at a constant rate. In other embodiments, the electrically charged first solution and the electrically charged second solution can both be discharged at variable rates. In some embodiments, the electrically charged first solution can be discharged at a constant rate and the electrically charged second solution can be discharged at a constant or variable rate. In some embodiments, the electrically charged first solution can be discharged at a constant rate and the electrically charged second solution can be discharged at a constant or variable rate that increases (e.g., continuously or in a stepwise fashion) as the electrospinning process proceeds.

In some cases, the electrically charged first solution can be discharged at a rate (e.g., a constant rate) of from 1 mL/hr to 8 mL/hr (e.g., at a rate of about 4 mL/hr). In some cases, the electrically charged second solution can be discharged at a constant or variable rate that increases (e.g., continuously or in a stepwise fashion) as the electrospinning process proceeds within the range of from 1 mL/hr to 8 mL/hr (e.g., within the range of from 1 mL/hr to 4 mL/hr).

Once the electrospun scaffold is formed, the particles comprising the porogen can be removed from amongst the nanofibers using any suitable method. For example, in some cases, the porogen particles can be dissolved by contacted the electrospun scaffold with a solvent for the porogen (and non-solvent for the nanofibers). By way of example, in some cases, the porogen can comprise a water-soluble substance, such as a water-soluble polymer. In some cases, the porogen can comprise an alcohol-soluble substance, such as a polymer that is soluble in an alcohol such as methanol or ethanol. In certain embodiments, the porogen can comprise polyethylene glycol (PEG), also known as polyethylene oxide (PEO). Methods of removing the porogen can comprise, for example, contacting the porogen particles (e.g., the PEG particles) with a solvent such as ethanol, water, or a combination thereof.

By delivering a porogen during the electrospinning process, the porosity of the electrospun scaffold can be substantially increased relative to an analogous scaffold prepared without the use of a porogen. According to the claimed invention, the tubular matrix has a porosity of at least 50% (e.g., a porosity of from 50% to 90%, or a porosity of from 70% to 90%), as determined by mercury porosimetry or apparent density. The amount of porogen relative to the polymer blend can be varied. In some embodiments, the weight ratio of porogen to the polymer blend of biodegradable synthetic polymer and biopolymer can be from 1:1 to 20:1 (e.g. from 5:1 to 20:1; from 10:1 to 20:1; from 1:1 to 15:1; or from 5:1 to 15:1). In some embodiments, the porogen is delivered via an electrospraying process.

The dimensions of the electrospun scaffold can be varied based on the desired dimensions of the electrospun scaffold (and by extension the resulting graft). In some cases, the tubular matrix can have a wall thickness of from 500 microns to 1500 microns (e.g., from 500 microns to 1000 microns, or from 500 microns to 750 microns). In some cases, the lumen of the electrospun can have a diameter of from 2.5 mm to 6.0 mm (e.g., from 3.0 mm to 5.5 mm).

Extracellular matrix-producing cells (e.g., fibroblasts or smooth muscle cells) are then cultured on the electrospun scaffold in a bioreactor to form the tissue-engineered tubular construct. The tissue-engineered tubular construct can be conditioned during the culturing step within the bioreactor by moving a fluid through the lumen as a pulsed flow. In some embodiments, a stretchable tubing extends through the lumen, and the graft is conditioned by expanding the stretchable tubing using a pulsatile flow or compressed fluid (e.g., using a compressed gas to compress a fluid within the stretchable tubing). The diameter of the stretchable tubing can be expanded by from about 2% to about 15% to condition the overlying tubular construct. The pulse frequency can range from 0 to 200 cycles per second. Once formed, the tissue-engineered tubular construct is decellularized to form the tissue-engineered scaffold. The decellularization process leaves behind a coating of cellularly-deposited extracellular matrix.

Endothelial cells from the subject are then cultured on the decellularized tissue-engineered scaffold to form a graft (e.g., autologous vascular grafts) for implantation into a subject. The endothelial cells can be cultured on the decellularized tissue-engineered scaffold in a bioreactor to form the graft. This can comprise seeding endothelial cells from the subject on the interior surface of the tissue-engineered scaffold. The endothelial cells can be obtained from any suitable source. For example, the endothelial cells can comprise cells obtained from subcutaneous fat, cells obtained from veins, cells cultured from peripheral blood circulating cells, and combinations thereof. Optionally, the tissue-engineered scaffold can be treated with an agent to facilitate adhesion of the endothelial cells, such as fibronectin.

The graft can be conditioned during the culturing step within the bioreactor by moving a fluid through the lumen of the graft as a pulsed flow. The pulsed flow can comprise flow at a variable rate (e.g., a rate that increases over time continuously or in a stepwise fashion) to induce a wall shear stress. The pulsed flow can comprise flow at a variable rate (e.g., a rate that increases over time continuously or in a stepwise fashion) to induce a wall shear stress of from 1 dyne/cm² to 30 dyne/cm². In some embodiments, a stretchable tubing extends through the lumen, and the graft is conditioned by moving compressed fluid through the stretchable tubing. The compressed fluid stretches the diameter of the tubing by 2% to 15% to condition the overlying tubular construct. The pulse frequency can range from 0 to 200 cycles per second.

Also provided are electrospun scaffolds that comprise a substantially tubular matrix formed from nanofibers that comprise a blend of a biodegradable synthetic polymer and a biopolymer. The tubular matrix comprises an external surface, an internal surface, and a lumen extending therethrough. The nanofibers can comprise from 60-80% by weight biodegradable synthetic polymer and from 10 -40% by weight biopolymer. The tubular matrix can have a porosity of at least 50% (e.g., a porosity of from 70% to 90%), as determined by mercury porosimetry or apparent density.

The nanofibers can comprise from 60-80% by weight biodegradable synthetic polymer and from 10-40% by weight biopolymer. The biodegradable synthetic polymer can comprise, for example, a polyester such as polylactic acid (PLA), polyglycolic acid (PGA), poly lactic-co-glycolide (PLGA), polycaprolactone (PCL), polydioxanone (PDS), a polyhydroxyalkanoate (PHA), polyurethane (PU), copolymers thereof, and blends thereof. Other suitable biodegradable synthetic polymers include, for example, polyurethanes. In certain embodiments, the biodegradable synthetic polymer can comprise PGA. The biopolymer can comprise, for example, a peptide or protein such as gelatin, collagen, elastin, silk fibroin, and combinations thereof. In certain embodiments, the biodegradable synthetic polymer can comprise gelatin. In some embodiments, the biodegradable synthetic polymer comprises polyglycolic acid (PGA) and the biopolymer comprises gelatin. The nanofibers have a diameter of from 50 nm to 2000 nm. e.g., from 50 nm to 1000 nm, from 50 nm to 500 nm, from 500 nm to 2000 nm, or from 500 nm to 1000 nm). In some cases, the electrospun scaffold can be substantially free of crosslinkers such as glutaraldehyde.

The dimensions of the electrospun scaffold can be varied based on the desired dimensions of the electrospun scaffold (and by extension the resulting graft). In some cases, the tubular matrix can have a wall thickness of from 500 microns to 1500 microns (e.g., from 500 microns to 1000 microns, or from 500 microns to 750 microns). In some cases, the lumen of the electrospun can have a diameter of from 2.5 mm to 6.0 mm (e.g., from 3.0 mm to 5.5 mm).

Also provided are methods of making electrospun scaffolds. Methods of making electrospun scaffolds can comprise (i) electrically charging a first solution comprising a biodegradable synthetic polymer and a biopolymer, wherein the first solution comprises from 60-80% by weight biodegradable synthetic polymer and from 10-40% by weight biopolymer; (ii) electrically charging a second solution comprising a porogen; (iii) discharging the electrically charged first solution and the electrically charged second solution onto a grounded target under an electrostatic field, such that the movement of the electrically charged first solution under the electrostatic field causes the electrically charged first solution to evaporate and produce nanofibers comprising a blend of the biodegradable synthetic polymer and the biopolymer on the grounded target, and the movement of the electrically charged second solution under the electrostatic field causes the electrically charged second solution to evaporate and produce particles comprising the porogen amongst the nanofibers on the grounded target; and (iv) removing the particles comprising the porogen from amongst the nanofibers to form the electrospun scaffold.

The biodegradable synthetic polymer can comprise, for example, a polyester such as polylactic acid (PLA), polyglycolic acid (PGA), poly lactic-co-glycolide (PLGA), polycaprolactone (PCL), polydioxanone (PDS), a polyhydroxyalkanoate (PHA), polyurethane (PU), copolymers thereof, and blends thereof. Other suitable biodegradable synthetic polymers include, for example, polyurethanes. In certain embodiments, the biodegradable synthetic polymer can comprise PGA. The biopolymer can comprise, for example, a peptide or protein such as gelatin, collagen, elastin, silk fibroin, and combinations thereof. In certain embodiments, the biodegradable synthetic polymer can comprise gelatin. In some embodiments, the biodegradable synthetic polymer comprises polyglycolic acid (PGA) and the biopolymer comprises gelatin.

In some embodiments, the grounded target can comprise a rotating mandrel. The dimensions of the mandrel and the parameters of the electrospinning process can be varied based on the desired dimensions of the electrospun scaffold (and by extension the resulting graft).

In some embodiments, the electrically charged first solution and the electrically charged second solution can both be discharged at a constant rate. In other embodiments, the electrically charged first solution and the electrically charged second solution can both be discharged at variable rates. In some embodiments, the electrically charged first solution can be discharged at a constant rate and the electrically charged second solution can be discharged at a variable rate. In some embodiments, the electrically charged first solution can be discharged at a constant rate and the electrically charged second solution can be discharged at a variable rate that increases (e.g., continuously or in a stepwise fashion) as the electrospinning process proceeds.

In some cases, the electrically charged first solution can be discharged at a rate (e.g., a constant rate) of from 1 mL/hr to 8 mL/hr (e.g., at a rate of about 4 mL/hr). In some cases, the electrically charged second solution can be discharged at a variable rate that increases (e.g., continuously or in a stepwise fashion) as the electrospinning process proceeds within the range of from 1 mL/hr to 8 mL/hr (e.g., within the range of from 1 mL/hr to 4 mL/hr).

Once the electrospun scaffold is formed, the particles comprising the porogen can be removed from amongst the nanofibers using any suitable method. For example, in some cases, the porogen particles can be dissolved by contacted the electrospun scaffold with a solvent for the porogen (and non-solvent for the nanofibers). By way of example, in some cases, the porogen can comprise a water-soluble substance, such as a water-soluble polymer. In some cases, the porogen can comprise an alcohol-soluble substance, such as a polymer that is soluble in an alcohol such as methanol or ethanol. In certain embodiments, the porogen can comprise polyethylene glycol (PEG). Methods of removing the porogen can comprise, for example, contacting the porogen particles (e.g., the PEG particles) with a solvent such as ethanol, water, or a combination thereof.

By delivering a porogen during the electrospinning process, the porosity of the electrospun scaffold can be substantially increased relative to an analogous scaffold prepared without the use of a porogen. According to the claimed invention, the tubular matrix has a porosity of at least 50% (e.g., a porosity of from 50% to 90%, or a porosity of from 70% to 90%), as determined by mercury porosimetry or apparent density. The amount of porogen relative to the polymer blend can be varied. In some embodiments, the weight ratio of porogen to the polymer blend of biodegradable synthetic polymer and biopolymer can be from 1:1 to 20:1 (e.g. from 5:1 to 20:1; from 10:1 to 20:1; from 1:1 to 15:1; or from 5:1 to 15:1). In some embodiments, the porogen is delivered via an electrospraying process.

Also described herein are tissue-engineered scaffolds prepared by a process as defined in the claims.

The nanofibers can comprise from 60-80% by weight biodegradable synthetic polymer and from 10-40% by weight biopolymer. The biodegradable synthetic polymer can comprise, for example, a polyester such as polylactic acid (PLA), polyglycolic acid (PGA), poly lactic-co-glycolide (PLGA), polycaprolactone (PCL), polydioxanone (PDS), a polyhydroxyalkanoate (PHA), polyurethane (PU), copolymers thereof, and blends thereof. Other suitable biodegradable synthetic polymers include, for example, polyurethanes. In certain embodiments, the biodegradable synthetic polymer can comprise PGA. The biopolymer can comprise, for example, a peptide or protein such as gelatin, collagen, elastin, silk fibroin, and combinations thereof. In certain embodiments, the biodegradable synthetic polymer can comprise gelatin. In some embodiments, the biodegradable synthetic polymer comprises polyglycolic acid (PGA) and the biopolymer comprises gelatin. The nanofibers have a diameter of from 50 nm to 2000 nm. e.g., from 50 nm to 1000 nm, from 50 nm to 500 nm, from 500 nm to 2000 nm, or from 500 nm to 1000 nm). In some cases, the electrospun scaffold can be substantially free of crosslinkers such as glutaraldehyde.

The electrospun scaffold can be prepared by a method that comprises electrically charging a first solution comprising the biodegradable synthetic polymer and the biopolymer; electrically charging a second solution comprising a porogen; discharging the electrically charged first solution and the electrically charged second solution onto a grounded target (e.g., a rotating mandrel) under an electrostatic field, such that the movement of the electrically charged first solution under the electrostatic field causes the electrically charged first solution to evaporate and produce the nanofibers that form the tubular matrix on the grounded target, and the movement of the electrically charged second solution under the electrostatic field causes the electrically charged second solution to evaporate and produce particles comprising the porogen amongst the nanofibers on the grounded target; and removing the particles comprising the porogen from amongst the nanofibers to form the electrospun scaffold.

In some embodiments, the grounded target can comprise a rotating mandrel. The dimensions of the mandrel and the parameters of the electrospinning process can be varied based on the desired dimensions of the electrospun scaffold (and by extension the resulting graft).

In some embodiments, the electrically charged first solution and the electrically charged second solution can both be discharged at a constant rate. In other embodiments, the electrically charged first solution and the electrically charged second solution can both be discharged at variable rates. In some embodiments, the electrically charged first solution can be discharged at a constant rate and the electrically charged second solution can be discharged at a variable rate. In some embodiments, the electrically charged first solution can be discharged at a constant rate and the electrically charged second solution can be discharged at a variable rate that increases (e.g., continuously or in a stepwise fashion) as the electrospinning process proceeds.

In some cases, the electrically charged first solution can be discharged at a rate (e.g., a constant rate) of from 1 mL/hr to 8 mL/hr (e.g., at a rate of about 4 mL/hr). In some cases, the electrically charged second solution can be discharged at a constant or variable rate that increases (e.g., continuously or in a stepwise fashion) as the electrospinning process proceeds within the range of from 1 mL/hr to 8 mL/hr (e.g., within the range of from 1 mL/hr to 4 mL/hr).

Once the electrospun scaffold is formed, the particles comprising the porogen can be removed from amongst the nanofibers using any suitable method. For example, in some cases, the porogen particles can be dissolved by contacted the electrospun scaffold with a solvent for the porogen (and non-solvent for the nanofibers). By way of example, in some cases, the porogen can comprise a water-soluble substance, such as a water-soluble polymer. In some cases, the porogen can comprise an alcohol-soluble substance, such as a polymer that is soluble in an alcohol such as methanol or ethanol. In certain embodiments, the porogen can comprise polyethylene glycol (PEG). Methods of removing the porogen can comprise, for example, contacting the porogen particles (e.g., the PEG particles) with a solvent such as ethanol, water, or a combination thereof.

By utilizing a porogen during the electrospinning process, the porosity of the electrospun scaffold can be substantially increased relative to an analogous scaffold prepared without the use of a porogen. The tubular matrix has a porosity of at least 50% (e.g., a porosity of from 50% to 90%, or a porosity of from 70% to 90%), as determined by mercury porosimetry or apparent density. The amount of porogen relative to the polymer blend can be varied. In some embodiments, the weight ratio of porogen to the polymer blend of biodegradable synthetic polymer and biopolymer can be from 1:1 to 20:1 (e.g. from 5:1 to 20:1; from 10:1 to 20:1; from 1:1 to 15:1; or from 5:1 to 15:1).

The dimensions of the electrospun scaffold can be varied based on the desired dimensions of the electrospun scaffold (and by extension the resulting graft). In some cases, the tubular matrix can have a wall thickness of from 500 microns to 1500 microns (e.g., from 500 microns to 1000 microns, or from 500 microns to 750 microns). In some cases, the lumen of the electrospun can have a diameter of from 2.5 mm to 6.0 mm (e.g., from 3.0 mm to 5.5 mm).

Extracellular matrix-producing cells (e.g., fibroblasts or smooth muscle cells) are then cultured on the electrospun scaffold in a bioreactor to form the tissue-engineered tubular construct. The tissue-engineered tubular construct can be conditioned during the culturing step within the bioreactor by moving a fluid through the lumen as a pulsed flow. Once formed, the tissue-engineered tubular construct are decellularized to form the tissue-engineered scaffold.

In some embodiments, the tissue-engineered scaffold can comprise at least some residual biodegradable synthetic polymer. For example, the tissue-engineered scaffold can comprise at least 0.5% by weight (e.g., from 0.5% to 50% by weight, or from 5% to 50% by weight) residual biodegradable synthetic polymer, based on the total weight of the scaffold. In some of these embodiments, the residual biodegradable synthetic polymer can comprise a polyester, such as polylactic acid (PLA), polyglycolic acid (PGA), poly lactic-co-glycolide (PLGA), polycaprolactone (PCL), polydioxanone (PDS), a polyhydroxyalkanoate (PHA), polyurethane (PU), copolymers thereof, and blends thereof. In certain embodiments, the residual biodegradable synthetic polymer comprises polycaprolactone (PCL).

Also provided are methods of forming grafts (e.g., autologous vascular grafts) for implantation into a subject that comprise culturing endothelial cells from the subject on the tissue-engineered scaffolds described above to form a graft for implantation into the subject. The endothelial cells can be cultured on the decellularized tissue-engineered scaffold in a bioreactor. This can comprise seeding endothelial cells from the subject on the interior surface of the tissue-engineered scaffold. The endothelial cells can be obtained from any suitable source. For example, the endothelial cells can comprise cells obtained from subcutaneous fat, cells obtained from veins, cells cultured from peripheral blood circulating cells, and combinations thereof. Optionally, the tissue-engineered scaffold can be treated with an agent to facilitate adhesion of the endothelial cells, such as fibronectin.

The graft can be conditioned during the culturing step within the bioreactor by moving a fluid through the lumen of the graft as a pulsed flow. The pulsed flow can comprise flow at a variable rate (e.g., a rate that increases over time continuously or in a stepwise fashion) to induce a wall shear stress. The pulsed flow can comprise flow at a variable rate (e.g., a rate that increases over time continuously or in a stepwise fashion) to induce a wall shear stress of from 1 dyne/cm² to 30 dyne/cm².

### DESCRIPTION OF DRAWINGS

Figure 1 is a schematic illustration of an electrospinning process used to fabricate example tubular electrospun scaffolds.
Figure 2 is a schematic illustration of two example methods for culturing a population of extracellular matrix-producing cells on the electrospun tubular scaffold to form a tissue-engineered tubular construct.

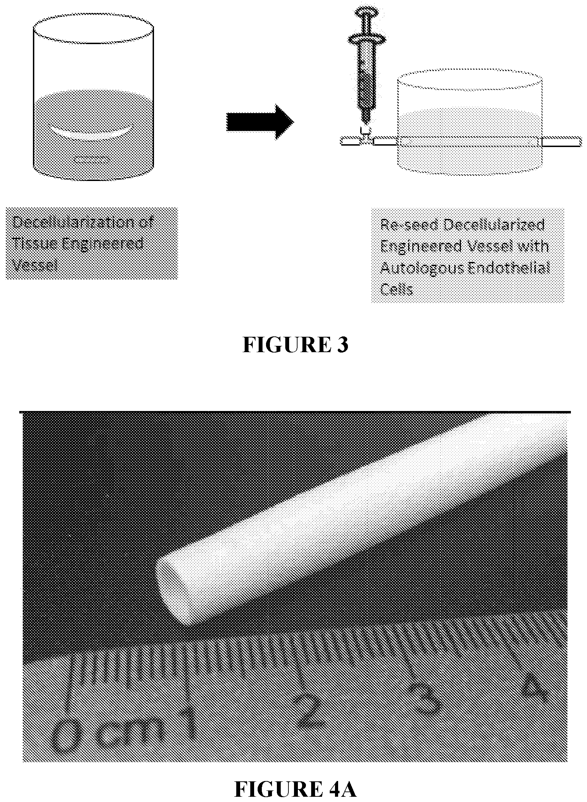

Figure 3 is a schematic illustration of methods for preparing a graft from the tissue-engineered tubular construct, including the steps of decellularizing the tissue-engineered tubular construct to form a tissue-engineered scaffold and culturing endothelial cells on the tissue-engineered scaffold to form the graft.
Figure 4A is a photo of an example electrospun tubular scaffold.

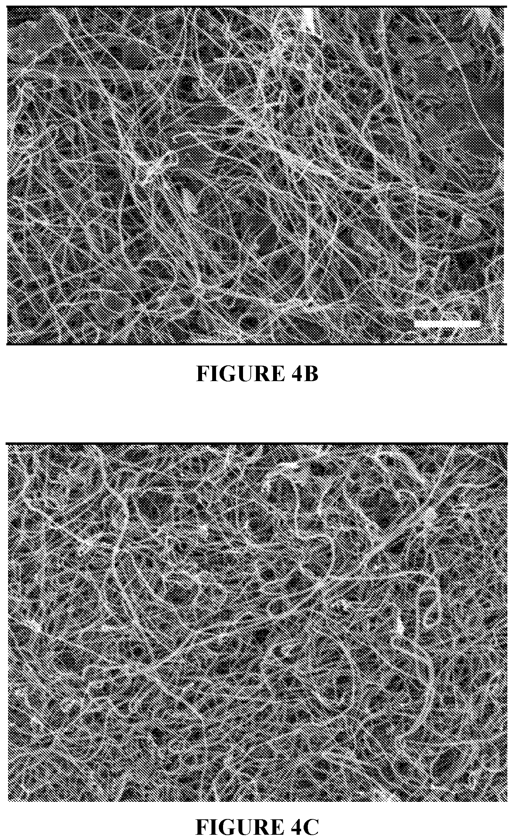

Figure 4B is a scanning electron microscopy (SEM) image of the electrospun tubular scaffold prior to removal of the particles comprising the porogen from amongst the nanofibers.
Figure 4C is a scanning electron microscopy (SEM) image of the electrospun tubular scaffold following removal of the particles comprising the porogen from amongst the nanofibers.

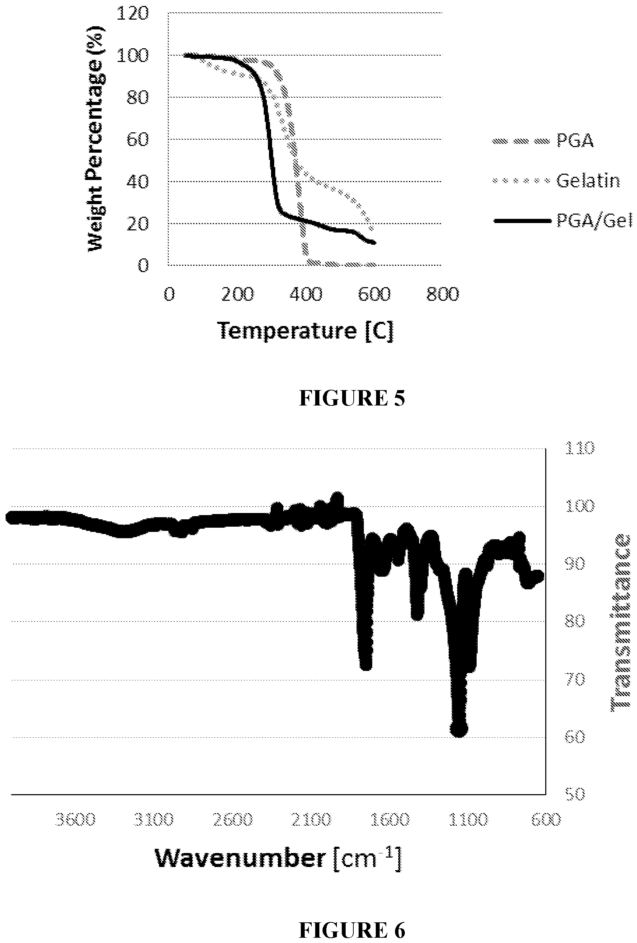

Figure 5 is a plot showing the thermogravimeteric analysis (TGA) of the electrospun tubular scaffold.
Figure 6 is a plot showing the Fourier transform infrared (FTIR) spectrum of the electrospun tubular scaffold.

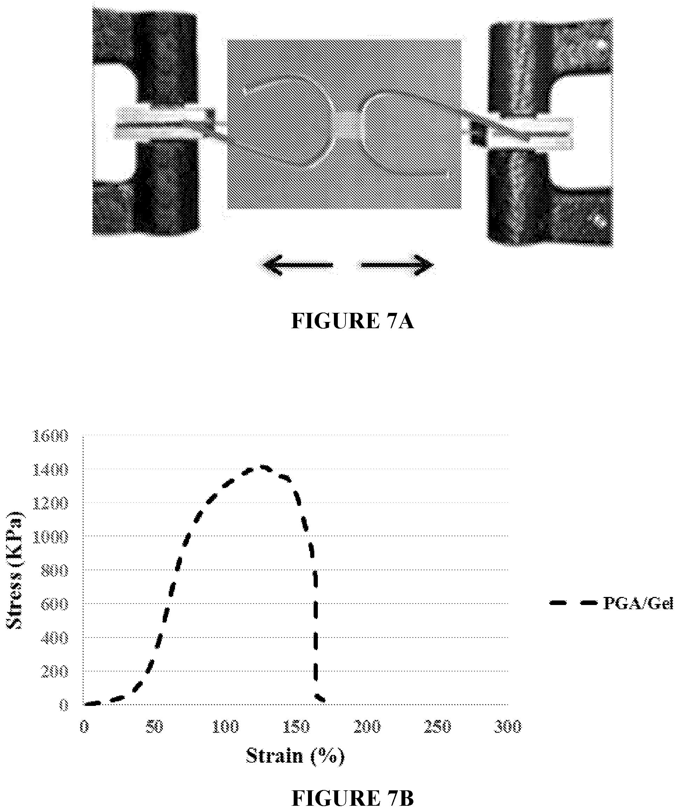

Figure 7A is a photo illustrating the Instron device used to perform uniaxial tensile tests of the electrospun tubular scaffold. To simulate radial force, a ringlet of the electrospun tubular scaffold spanned two hooks and the force was recorded until the scaffold failed.
Figure 7B is a plot illustrating the results of a uniaxial tensile test performed on the electrospun tubular scaffold. The ultimate tensile strength of the scaffold is the point of failure, and was around 1400 KPa.

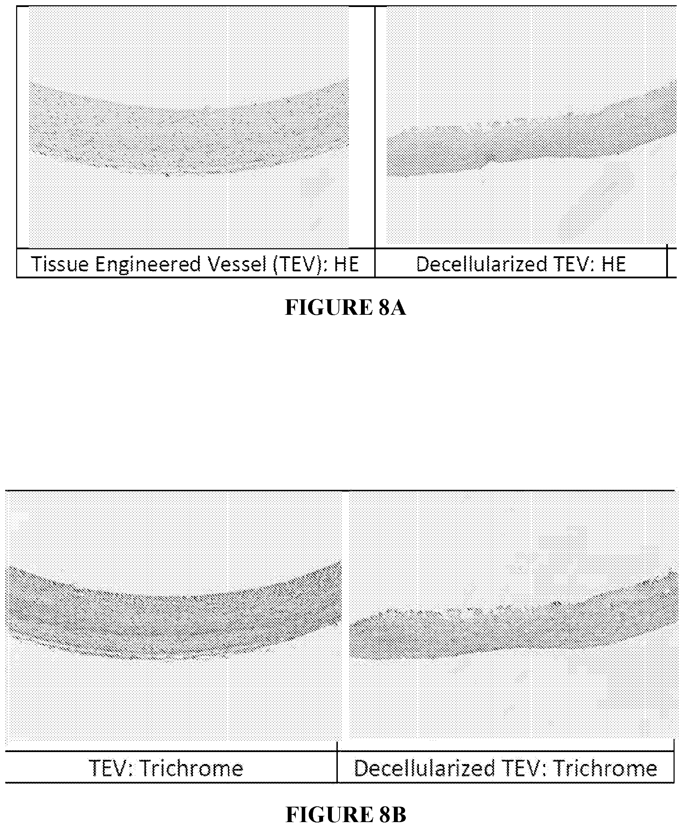

Figure 8A includes microscopy images of the tissue-engineered tubular construct (left panel) and the decellularized tissue-engineered scaffold (right panel) under hematoxylin and eosin (HE) staining.
Figure 8B includes microscopy images of the tissue-engineered tubular construct (left panel) and the decellularized tissue-engineered scaffold (right panel) under trichrome staining.

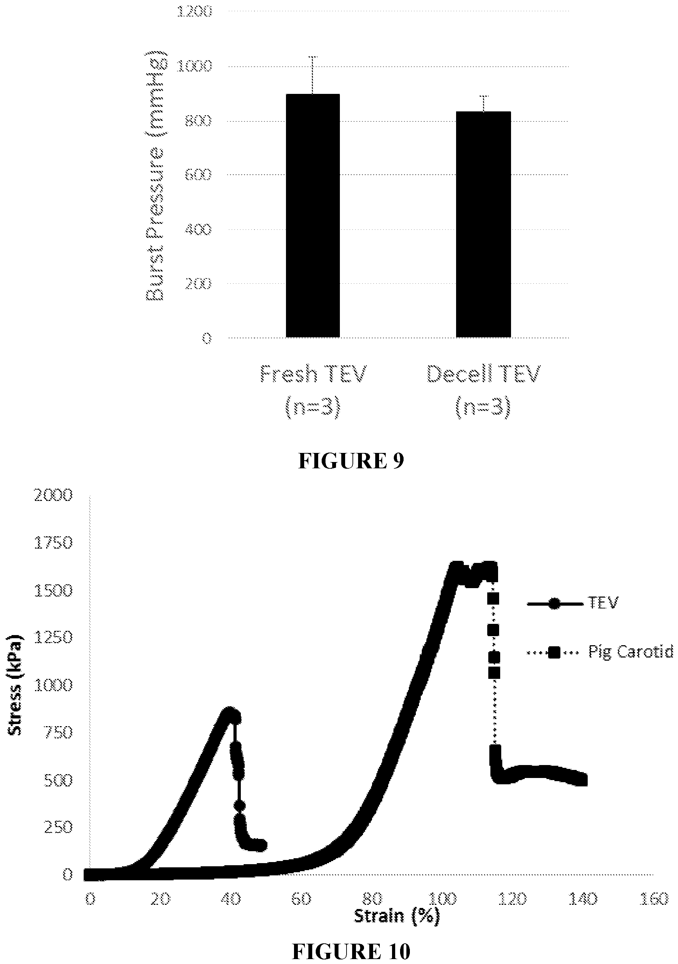

Figure 9 is a plot showing the burst pressure (in mmHg) of the tissue-engineered tubular construct (left bar) and the decellularized tissue-engineered scaffold (right bar). There was only a slight decrease in burst pressure after decellularization. The burst pressure was approximately 850 mm Hg after decellularization
Figure 10 is a plot illustrating the results of a uniaxial tensile test performed on the tissue-engineered tubular construct as well as a segment of pig carotid artery.
Figure 11A is a schematic top view of an example bioreactor.
Figure 11B is a schematic side view of an example bioreactor.
Figure 11C is a schematic traverse view of an example bioreactor.
Figure 12 is a schematic side view of an example bioreactor

### DETAILED DESCRIPTION

### Definitions

The term "subject," as used herein, is intended to include living organisms in which an immune response is elicited. Preferred subjects are mammals. Examples of subjects include but are not limited to, humans, monkeys, dogs, cats, mice, rates, cows, horses, pigs, goats and sheep.

The term "decellularized" or "decellularization," as used herein, refers to a structure from which the cellular and tissue content has been removed leaving behind an intact acellular infrastructure. The process of decellularization removes specialized tissue and cells from the structure, leaving behind a complex three-dimensional network of connective tissue. The connective tissue infrastructure is primarily composed of collagen. The decellularized structure provides a biocompatible substrate onto which different cell populations can be infused. Decellularized biostructures can be rigid, or semi-rigid, having an ability to alter their shapes.

The term "electrospinning" or "electrospun," as used herein, refers to methods where materials are streamed, sprayed, sputtered, dripped, or otherwise transported in the presence of an electric field. The electrospun material can be deposited from the direction of a charged container towards a grounded target, or from a grounded container in the direction of a charged target. In particular, the term "electrospinning" means a process in which fibers are formed from a charged solution comprising at least one biodegradable synthetic polymer and at least one biopolymer by streaming the electrically charged solution through an opening or orifice towards a grounded target. The terms "solution" and "fluid" is used in the context of producing an electrospun matrix and describes a liquid that is capable of being charged.

The term "preconditioning chamber," as used herein, refers to a container that allows a scaffold seeded with cells to be conditioned such that the cells on the scaffold develop under physiological conditions. For example, to create blood vessels, a scaffold can be seeded with endothelial cells and the endothelial cells allowed to develop under native fluid conditions such as pulsed conditions that mimic the pulse rate of blood through native vessels, or fluid flow conditions with alterations in pressure. To begin with, the pulse rate and the flow rate can be slow until the cells adjust to this pulse rate or flow rate, the flow rate and pulse rate can then gradually be increased until the cells adjust to the new pulse rate and flow rate and so forth. By gradually increasing the pulse rate and the flow rate, the vessels become conditioned to being able to withstand pressure as high as those produced during each heartbeat.

The biological fluid can be moved through the inside surface of the attached scaffold (lumen) or through a tube placed within the lumen as a continuous flow, for example at a flow rate that can be incremented over time to induce a wall shear in the range of about 1 dyne/cm² to about 30 dynes/cm². The pulse-rate can be incremented over time to induce a wall pressure distribution in the engineered blood vessel in the range of about 60 to about 200 mm Hg. A different of the same biological fluid can also be used to precondition the outside of the matrix.

The term "biological fluid" as used herein refers a liquid that can be used to precondition an engineered blood vessel. The biological fluid has a composition and viscosity that mimics blood so that the engineered blood vessels are exposed to the same fluid flow dynamics as native blood vessels. Examples of biological fluids can include any buffer, medium of physiological fluid (e.g., DMEM with 10% FCS with a blood viscosity). The viscosity of the fluids can be altered by adding high molecular weight proteins such as 100 kDa dextran. Other molecular weight dextrans can also be used. It will be appreciated that the amount of dextran to be used depends on the molecular weight and can range from about 10%, 20%, 30%, 40%, 50%, and 60%. The composition may also be varied by adding other blood like constituents such as salts

### Methods

Provided herein are methods of forming grafts (e.g., autologous vascular grafts, biliary conduits, ureter conduits, etc.) for implantation into subjects in need thereof. The methods employ an electrospun scaffold formed from nanofibers that comprise a blend of a biodegradable synthetic polymer and a biopolymer. Extracellular matrix-producing cells (e.g., fibroblasts) are then cultured on the electrospun scaffold in a bioreactor to form a cellular, tubular structure. The tubular structure is then decellularized, and endothelial cells obtained from the subject are cultured on the resulting decellularized scaffold to form a graft (e.g., autologous vascular grafts) for implantation into a subject. Because decellularized scaffolds can be prepared in advance, the methods described herein can be used to prepare autologous grafts for implantation in a subject in a relatively short, clinically relevant timeframe (e.g., from one week to one month).

The claimed method of forming grafts for implantation into a subject comprises (i) forming an electrospun scaffold comprising a substantially tubular matrix formed from nanofibers that comprise a blend of a biodegradable synthetic polymer and a biopolymer, wherein the tubular matrix comprises an external surface, an internal surface, and a lumen extending therethrough, wherein the nanofibers have a diameter of from 50 nm to 2000 nm, wherein the tubular matrix has a porosity of at least 50%, as determined by mercury porosimetry or apparent density; (ii) culturing a population of extracellular matrix-producing cells on the electrospun tubular scaffold to form a tissue-engineered tubular construct; (iii) decellularizing the tissue-engineered tubular construct to form a tissue-engineered scaffold; and (iv) culturing endothelial cells from the subject on the tissue-engineered scaffold to form a graft for implantation into the subject.

### Electrospinning

The process of electrospinning generally involves the creation of an electrical field at the surface of a liquid. The resulting electrical forces create a jet of liquid which carries electrical charge. The liquid jets may be attracted to other electrically charged objects at a suitable electrical potential. As the jet of liquid elongates and travels, it will harden and dry. The hardening and drying of the elongated jet of liquid may be caused by cooling of the liquid, i.e., where the liquid is normally a solid at room temperature; evaporation of a solvent, e.g., by dehydration, (physically induced hardening); or by a curing mechanism (chemically induced hardening). The produced fibers are collected on a suitably located, oppositely charged target substrate.

The electrospinning apparatus can include an electrodepositing mechanism and a target substrate. The electrodepositing mechanism includes at least one container to hold the solution that is to be electrospun. The container has at least one orifice or nozzle to allow the streaming of the solution from the container. If there are multiple containers, a plurality of nozzles may be used. One or more pumps (e.g., a syringe pump) used in connection with the container can be used to control the flow of solution streaming from the container through the nozzle. The pump can be programmed to increase or decrease the flow at different points during electrospinning.

The electrospinning occurs due to the presence of a charge in either the orifices or the target, while the other is grounded. In some embodiments, the nozzle or orifice is charged and the target is grounded. Those of skill in the electrospinning arts will recognize that the nozzle and solution can be grounded and the target can be electrically charged.

The target can also be specifically charged or grounded along a preselected pattern so that the solution streamed from the orifice is directed into specific directions. The electric field can be controlled by a microprocessor to create an electrospun matrix having a desired geometry. The target and the nozzle or nozzles can be engineered to be movable with respect to each other thereby allowing additional control over the geometry of the electrospun matrix to be formed. The entire process can be controlled by a microprocessor that is programmed with specific parameters that will obtain a specific preselected electrospun matrix.

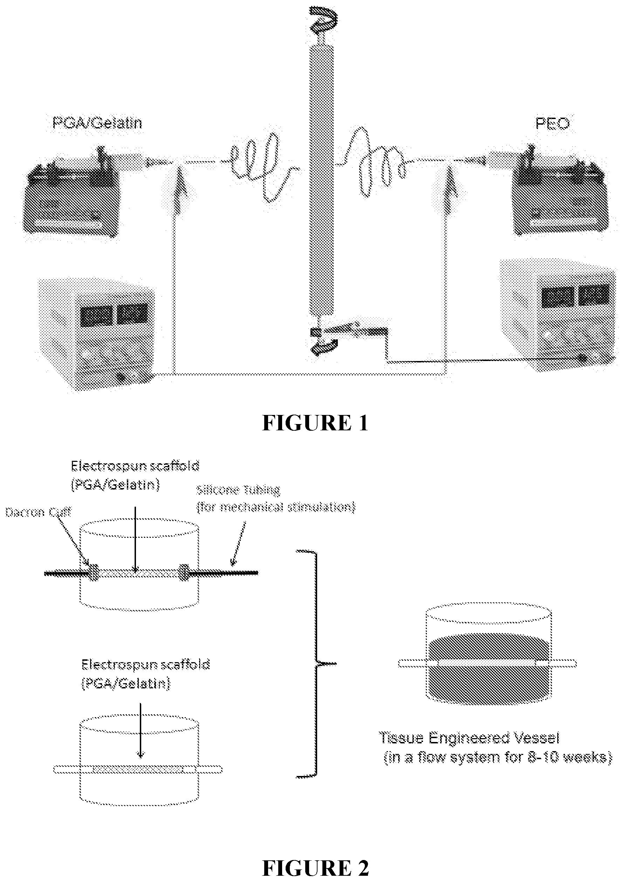

The process for preparing the electrospun scaffold is illustrated in Figure 1. Briefly, a first container (e.g., a syringe or micropipette) with an orifice or nozzle (e.g., a Taylor cone) is filled with a first solution comprising the biodegradable synthetic polymer and the biopolymer. A second container (e.g., a syringe or micropipette) with an orifice or nozzle (e.g., a Taylor cone) is filled with a second solution comprising the porogen. The first container and the second container are then suspended facing a grounded target, such as a metal ground rotating mandrel. The first container and the second container can be positioned so as to discharge onto different portions of the grounded target. For example, as shown in Figure 1, the first container and the second container can be positioned so as to discharge onto opposing sides of a rotating grounded target. High voltage is applied to the nozzle by a clamp to charge the solution. At a specific voltage determined for each solution, the solution in the container nozzle is directed towards the grounded target. The single jet stream of materials forms a splayed jet, upon reaching the grounded target, e.g., a rapidly rotating mandrel. The splayed jet collects and dries to form a three-dimensional, ultrathin, interconnected matrix of electrospun fibers comprising the biodegradable synthetic polymer and the biopolymer. The particles comprising the porogen are formed amongst the nanofibers on the grounded target (for example, using electrospraying techniques). The porogen particles can then be removed from amongst the nanofibers using any suitable method.

Minimal electrical current is involved in the electrospinning process, therefore the process does not denature the materials that form the electrospun matrix, because the current causes little or no temperature increase in the solutions during the procedure.

The electrospinning process can be manipulated to meet the specific requirements for any given application of the electrospun matrix. In one embodiment, a syringe can be mounted on a frame that moves in the x, y and z planes with respect to the grounded substrate. In another embodiment, a syringe can be mounted around a grounded substrate, for instance a tubular mandrel. In this way, the materials that form the matrix streamed from the syringe can be specifically aimed or patterned. Although the micropipette can be moved manually, the frame onto which the syringe is mounted can also be controlled by a microprocessor and a motor that allows the pattern of streaming to be predetermined. Such microprocessors and motors are known to one of ordinary skill in the art, for example matrix fibers can be oriented in a specific direction, they can be layered, or they can be programmed to be completely random and not oriented.

The degree of branching can be varied by many factors including, but not limited to, voltage (for example ranging from about 0 to 30,000 volts, such as from 14-16 kV), distance from a syringe tip to the substrate (for example from 1-100 cm, 0-40 cm, 10-15 cm, or 1-10 cm), the speed of rotation (for example, from 500 to 5000 RPM), the shape of the mandrel, the relative position of the a syringe tip and target (i.e. in front of, above, below, aside etc.), and the diameter of a syringe tip (approximately 0-2 mm), and the concentration and ratios of compounds that form the electrospun matrix. Other parameters which are important include those affecting evaporation of solvents such as temperature, pressure, humidity. The molecular weight of the polymer affects its ability to entangle and form fibers. Those skilled in the art will recognize that these and other parameters can be varied to form electrospun materials with characteristics that are particularly adapted for specific applications.

The biodegradable synthetic polymer can be any material prepared through a method of artificial synthesis, processing, or manufacture. Preferably the biodegradable synthetic polymer is biologically compatible. The biodegradable synthetic polymer can comprise, for example, a polyester such as polylactic acid (PLA), polyglycolic acid (PGA), poly lactic-co-glycolide (PLGA), polycaprolactone (PCL), polydioxanone (PDS), a polyhydroxyalkanoate (PHA), polyurethane (PU), copolymers thereof, and blends thereof. Examples of polyhydroxyalkanoates include poly-3-hydroxybutyrate (P3HB), poly-4-hydroxybutyrate (P4HB), polyhydroxyvalerate (PHV), polyhydroxyhexanoate (PHH), polyhydroxyoctanoate (PHO), copolymers thereof, and blends thereof. Other suitable biodegradable synthetic polymers include, for example, polyurethanes. In certain embodiments, the biodegradable synthetic polymer can comprise PGA.

In some embodiments, the nanofibers can comprise at least 60% by weight (e.g., at least 65% by weight, at least 70% by weight, or at least 75% by weight) biodegradable synthetic polymer, based on the total weight of the nanofiber. In some embodiments, the nanofibers can comprise 80% or less by weight (e.g., 75% or less by weight, 70% or less by weight, or 65% or less by weight) biodegradable synthetic polymer, based on the total weight of the nanofiber. The nanofibers can comprise an amount of biodegradable synthetic polymer ranging from any of the minimum values described above to any of the maximum values described above. For example, the nanofibers can comprise from 60-80% by weight (e.g., from 60-70% by weight, from 70-80% by weight, or from 65-75% by weight) biodegradable synthetic polymer, based on the total weight of the nanofiber.

The biopolymer can be a naturally occurring organic material including any material naturally found in the body of a mammal, plant, or other organism. The biopolymer can comprise, for example, a peptide or protein such as gelatin, collagen, elastin, silk fibroin, and combinations thereof. In certain embodiments, the biodegradable synthetic polymer can comprise gelatin.

In some embodiments, the nanofibers can comprise at least 10% by weight (e.g., at least 25% by weight, at least 30% by weight, or at least 30% by weight) biopolymer, based on the total weight of the nanofiber. In some embodiments, the nanofibers can comprise 40% or less by weight (e.g., 35% or less by weight, 30% or less by weight, or 25% or less by weight) biopolymer, based on the total weight of the nanofiber. The nanofibers can comprise an amount of biopolymer ranging from any of the minimum values described above to any of the maximum values described above. For example, the nanofibers can comprise from 10-40% by weight (e.g., from 20-30% by weight, from 30-40% by weight, or from 25-35% by weight) biopolymer, based on the total weight of the nanofiber.

In some embodiments, the nanofibers can comprise from 60-80% by weight (e.g., from 60-70% by weight, from 70-80% by weight, or from 65-75% by weight) biodegradable synthetic polymer and from 10-40% by weight (e.g., from 10-30% by weight, from 30-40% by weight, or from 25-35% by weight) biopolymer, based on the total weight of the nanofiber. In some embodiments, the biodegradable synthetic polymer comprises polyglycolic acid (PGA) and the biopolymer comprises gelatin.

The porogen can be any suitable materials, such as salts or other extractable agents, the dissolution of which will result in void space and increased porosity in the matrix. In some cases, the porogen can comprise a water-soluble substance, such as a water-soluble polymer. In some cases, the porogen can comprise an alcohol-soluble substance, such as a polymer that is soluble in an alcohol such as methanol or ethanol. In certain embodiments, the porogen can comprise polyethylene glycol (PEG), which is also known as polyethylene oxide (PEO). Once the electrospun scaffold is formed, the particles comprising the porogen can be removed from amongst the nanofibers using any suitable method. Methods of removing the porogen can comprise, for example, contacting the porogen particles (e.g., the PEG particles) with a solvent such as ethanol, water, or a combination thereof.

By delivering a porogen during the electrospinning process, the porosity of the electrospun scaffold can be substantially increased relative to an analogous scaffold prepared without the use of a porogen. In some embodiments, the porogen is delivered via an electrospraying process. According to the claimed invention, the tubular matrix has a porosity of at least 50% (e.g., at least 55%, at least 60%, at least 65%, at least 70%, at least 75%, at least 80%, or at least 85%), as determined by mercury porosimetry or apparent density. In some embodiments, the tubular matrix can have a porosity of 90% or less (e.g., 85% or less, 80% or less, 75% or less, 70% or less, 65% or less, 60% or less, or 55% or less), as determined by mercury porosimetry or apparent density. The tubular matrix can have a porosity ranging from any of the minimum values described above to any of the maximum values described above. For example, the tubular matrix can have a porosity ranging from 50% to 90% (e.g., from 60% to 90%, or from 70% to 90%), as determined by mercury porosimetry or apparent density. The amount of porogen added can be varied to influence the porosity of the resulting tubular matrix. In some embodiments, the weight ratio of porogen to the polymer blend of biodegradable synthetic polymer and biopolymer can be from 1:1 to 20:1 (e.g. from 5:1 to 20:1; from 10:1 to 20:1; from 1:1 to 15:1; or from 5:1 to 15:1).

The polymers to be electrospun can be present in the solution at any concentration that will allow electrospinning. In one embodiment, the polymers to be electrospun are present in the solution at concentrations between 0 and about 1.000 g/ml. In another embodiment, the polymers to be electrospun are present in the solution at total solution concentrations between 10-15 w/v % (100-150 mg/ml or 0-0.1 g/L).

The polymers can be dissolved in any solvent that allows delivery of the polymers to the orifice, tip of a syringe, under conditions that the polymers are electrospun. Solvents useful for dissolving or suspending a material or a substance will depend on the identity of the polymers. Electrospinning techniques often require more specific solvent conditions. For example, collagen and gelatin can be electrodeposited as a solution or suspension in water, 2,2,2-trifluoroethanol, 1,1,1,3,3,3-hexafluoro-2-propanol (also known as hexafluoroisopropanol or HFIP), or combinations thereof. Elastin can be electrodeposited as a solution or suspension in water, 2,2,2-trifluoroethanol, isopropanol, HFIP, or combinations thereof, such as isopropanol and water. Other lower order alcohols, especially halogenated alcohols, may be used. Other solvents that may be used or combined with other solvents in electrospinning polymers include acetamide, N-methylformamide, N,N-dimethylformamide (DMF), dimethylsulfoxide (DMSO), dimethylacetamide, N-methyl pyrrolidone (NMP), acetic acid, trifluoroacetic acid, ethyl acetate, acetonitrile, trifluoroacetic anhydride, 1,1,1-trifluoroacetone, maleic acid, hexafluoroacetone. Organic solvents such as methanol, chloroform, and trifluoroethanol (TFE) and emulsifying agents can also be used.

The selection of a solvent is based in part on consideration of secondary forces that stabilize polymer-polymer interactions and the solvent's ability to replace these with strong polymer-solvent interactions. In the case of polypeptides such as collagen, and in the absence of covalent crosslinking, the principal secondary forces between chains are: (1) coulombic, resulting from attraction of fixed charges on the backbone and dictated by the primary structure (e.g., lysine and arginine residues will be positively charged at physiological pH, while aspartic or glutamic acid residues will be negatively charged); (2) dipole-dipole, resulting from interactions of permanent dipoles; the hydrogen bond, commonly found in polypeptides, is the strongest of such interactions; and (3) hydrophobic interactions, resulting from association of non-polar regions of the polypeptide due to a low tendency of non-polar species to interact favorably with polar water molecules. Therefore, solvents or solvent combinations that can favorably compete for these interactions can dissolve or disperse polypeptides. For example, HFIP and TFE possess a highly polar OH bond adjacent to a very hydrophobic fluorinated region. While not wanting to be bound by the following theories, it is believed that the alcohol portion can hydrogen bond with peptides, and can also solvate charges on the backbone, thus reducing Coulombic interactions between molecules. Additionally, the hydrophobic portions of these solvents can interact with hydrophobic domains in polypeptides, helping to resist the tendency of the latter to aggregate via hydrophobic interactions. It is further believed that solvents such as HFIP and TFE, due to their lower overall polarities compared to water, do not compete well for intramolecular hydrogen bonds that stabilize secondary structures such as an alpha helix. Consequently, alpha helices in these solvents are believed to be stabilized by virtue of stronger intramolecular hydrogen bonds. The stabilization of polypeptide secondary structures in these solvents is believed desirable, especially in the cases of collagen and elastin, to preserve the proper formation of collagen fibrils during electrospinning.

In one embodiment, the solvent has a relatively high vapor pressure to promote the stabilization of an electrospinning jet to create a fiber as the solvent evaporates. In embodiments involving higher boiling point solvents, it is often desirable to facilitate solvent evaporation by warming the spinning or spraying solution, and optionally the electrospinning stream itself, or by electrospinning in reduced atmospheric pressure. It is also believed that creation of a stable jet resulting in a fiber is facilitated by a high surface tension of the polymer/solvent mixture.

In some embodiments, the electrically charged first solution and the electrically charged second solution can both be discharged at a constant rate. In other embodiments, the electrically charged first solution and the electrically charged second solution can both be discharged at variable rates. In some embodiments, the electrically charged first solution can be discharged at a constant rate and the electrically charged second solution can be discharged at a variable rate. In some embodiments, the electrically charged first solution can be discharged at a constant rate and the electrically charged second solution can be discharged at a variable rate that increases (e.g., continuously or in a stepwise fashion) as the electrospinning process proceeds.

In some cases, the electrically charged first solution can be discharged at a rate (e.g., a constant rate) of from 1 mL/hr to 8 mL/hr (e.g., at a rate of about 4 mL/hr). In some cases, the electrically charged second solution can be discharged at a variable rate that increases (e.g., continuously or in a stepwise fashion) as the electrospinning process proceeds within the range of from 1 mL/hr to 8 mL/hr (e.g., within the range of from 1 mL/hr to 4 mL/hr).

The dimensions of the electrospun scaffold can be varied based on the desired dimensions of the electrospun scaffold (and by extension the resulting graft). In some cases, the tubular matrix can have a wall thickness of from 500 microns to 1500 microns (e.g., from 500 microns to 1000 microns, or from 500 microns to 750 microns). In some cases, the lumen of the electrospun can have a diameter of from 2.5 mm to 6.0 mm (e.g., from 3.0 mm to 5.5 mm).

The nanofibers have a diameter of from 50 nm to 2000 nm (e.g., from 50 nm to 1000 nm, from 50 nm to 500 nm, from 500 nm to 2000 nm, or from 500 nm to 1000 nm). In some cases, the electrospun scaffold can be substantially free of crosslinkers such as glutaraldehyde.

### Tissue-Engineered Tubular Constructs and Bioreactors

Extracellular matrix-producing cells (ECM-producing, e.g., fibroblasts or smooth muscle cells) can be cultured on the electrospun scaffold in a bioreactor to form the tissue-engineered tubular construct. For example, human dermal fibroblasts can be cultured on the electrospun scaffold in a bioreactor. During culture, the ECM-producing cells deposit ECM onto the fibers of the electrospun scaffold. The fibers of cellularly-deposited ECM are better aligned than, for example, fibers of ECM applied manually (e.g., via soaking in a solution containing ECM).

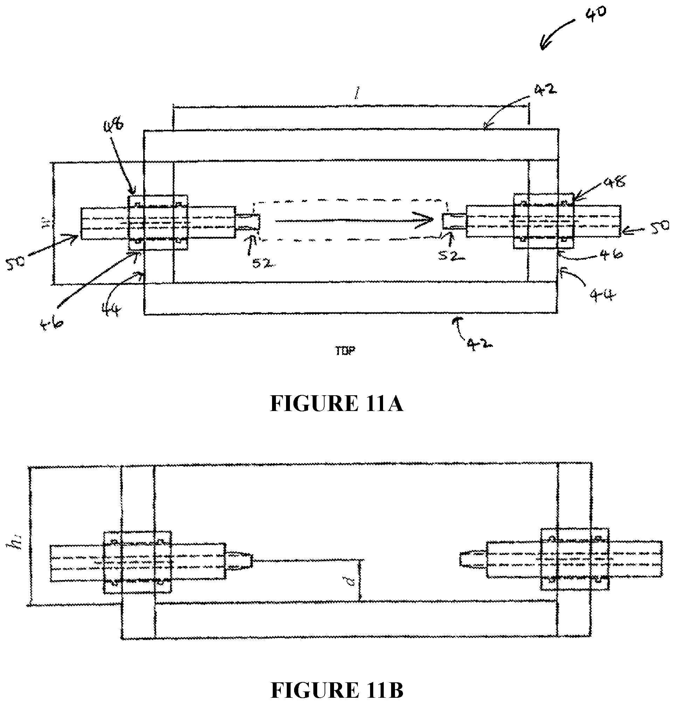

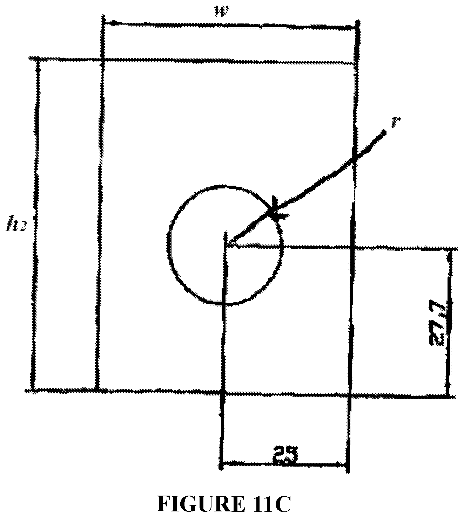

For example, the ECM-producing cells can be cultured on the electrospun scaffold in the bioreactor illustrated in Figures 11A-11C. The dimensions of the bioreactor chamber are such that it can hold a scaffold seeded with cells. Figure 11A shows the top view of one embodiment of the bioreactor chamber (also referred to as a preconditioning chamber) **40** with a first and second long wall **42** and a first and second short wall **44**. The first and second short walls **44** each have an opening **46** that accommodates a platform **48** that can hold a vessel **50** having an attachment element **52**. The scaffold seeded with cells (shown in hashed lines) can be attached to the first and second attachment elements **52** of the vessel **50**. The vessel is operatively linked to a fluid flow system (not shown) that can pump biological fluid through one end of the vessel **50**, through the attached tubular scaffold seeded with cells, and through the other end of the vessel in a continuous manner. The preconditioning chamber can be sterilized between uses. Sterilization can be accomplished by, for example, exposure to ethylene oxide gas. Figure 11B shows the side view of the bioreactor of Figure 11A and Figure 11C shows a traverse view Figure 11A. The dimensions shown on FIGS. 11A-11C are examples given for a particular embodiment, and can vary depending upon the needs of other embodiments. The length, *l*, of the interior preconditioning chamber embodiment, shown in FIG. 11A can be from about 80 to about 150 millimeters. The width, w, as measured across the top of the interior preconditioning chamber embodiment, can be from about 50 to about 100 millimeters as shown in FIGS. 11A and 11C. The height, *h₁*, of the interior preconditioning chamber is from about 50 to about 100 millimeters as shown in FIG. 11B. The height, *h₂*, of the entire bioreactor (including the bottom wall of the chamber) is 60-120 millimeters as shown in FIG. 11C. The radius, *r*, of platform **48** is 10-12 millimeters, as shown in FIG. 11C. The distance, d, from the center of attachment element **52** to the bottom of the interior preconditioning chamber can be from about 15 to about 50 millimeters, as shown in FIG. 11B.

The biological fluid can be pumped using any pumping mechanism such as a gear pump. The chamber can further comprise a rotation device that can be used to rotate the chamber at a desired angle for example, by 45°, 90°, 180°, and 360°. The rotation device can be manually operated or can be automated such that the chamber is rotated at a desired speed and at a desired time. In other embodiments, the chamber can be a multichambered and can house more than one scaffold. In other embodiments, both the inside and the outside of the seeded scaffold can be preconditioned using the preconditioning chamber. In such embodiments, the chamber is filled with a volume of preconditioning fluid that can cover the attached seeded matrix. The fluid flow of the biological fluid on the outside of the matrix can be controlled by the same or a separate mechanism than the fluid flow on the inside of the matrix. The biological fluid on the outside may be the same as the biological fluid on the inside. Alternatively, or the biological fluid on the outside may be the different than the biological fluid on the inside. The fluid flow parameters and be the same for the biological fluid on the inside and the outside, or can be different.

The walls of the preconditioning chamber can be made of any suitable material such as plexiglass, plastics and the like as long as the material does not react with a biological fluid. The biological fluid can be moved through the inside surface (lumen) of the attached matrix as a continuous flow, for example with a continuous flow-rate that can be incremented over time to induce a wall shear in the range of about 1 dyne/cm² to about 30 dynes/cm². The pulse-rate can be incremented over time to induce a wall pressure distribution in the engineered blood vessel in the range of about 60 to about 200 mmHg. A different of the same biological fluid can also be used to precondition the outside of the matrix.

The biological fluid can have a composition and viscosity that mimics blood so that the engineered blood vessels are exposed same fluid flow dynamics as native blood vessels. Examples of biological fluids can include any buffer, medium of physiological fluid (e.g., DMEM with 10% FCS) The viscosity of the fluids can be altered by adding high molecular weight proteins such as 100 kDa dextran. Other molecular weight dextrans can also be used. It will be appreciated that the amount of dextran to be used depends on the molecular weight and can range from about 10%, 20%, 30%, 40%, 50%, and 60%. The composition may also be varied by adding other blood like constituents such as salts.

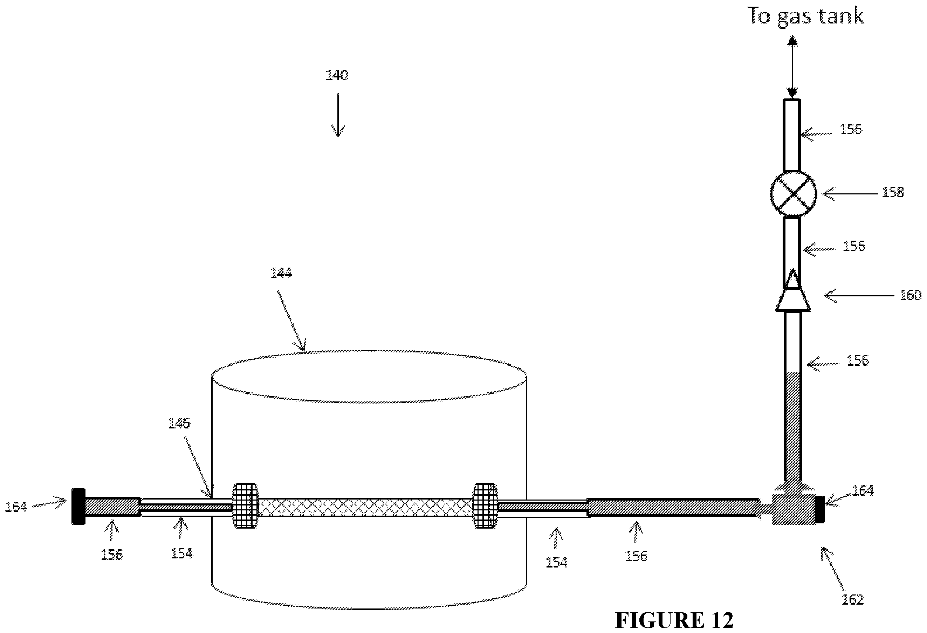

FIG. 12 shows an embodiment of a preconditioning device **140**. The rounded walls **144** each have openings **146** through which stretchable tubing **154** can be extended. The tubular scaffold seeded with cells can be positioned over stretchable tubing **154**. The stretchable tubing **154** is directly or indirectly linked to a fluid flow system at least partially filled with a compressed gas. The fluid flow system can be a hydraulic system that pumps compressed gas through one end of the stretchable tubing **154**, or into extension tubing **156** that connects with the stretchable tubing **154**. The stretchable tubing **154** contains a liquid in the portion that extends through the tubular scaffold, and the compressed gas is used to push the liquid against the walls of the stretchable tubing **154** in order to mechanically condition the tubular scaffold. The hydraulic system can control the pulse pressure and the pulse frequency at which the pressurized gas is delivered through the stretchable tubing **154** to mechanically condition the scaffold using, for example, pneumatic valve **158**. The preconditioning device **140** can optionally include an air filter **160**, a three-way valve **162**, and caps **164**. In some embodiments, a separate fluid flow system can be utilized to deliver a biological fluid to the preconditioning chamber to cover the exterior surfaces of the tubular scaffold seeded with cells.

Advantageously, the pressure and the frequency at which the pressurized gas is pulsed can be controlled as separate variables. The ability to separately control the pulse pressure and frequency during mechanical conditioning can be particularly advantageous for the engineering of blood vessels due to natural variation of these variables *in vivo.* For example, the pulse pressure delivered through stretchable tubing **154** can be adjusted to achieve a stretch of from about 2% to about 15% of the original diameter of the tubing **154**, including about 2%, about 3%, about 4%, about 5%, about 6%, about 7%, about 8%, about 9%, about 10%, about 11%, about 12%, about 13%, about 14%, and about 15%. Tubing **154** can be formed of any stretchable material (e.g., silicone or other elastic polymers). The hydraulic system can include, for example, a pneumatic valve **158** that controls the frequency at which the pressurized gas is delivered. The pulse frequency at which the pressurized gas is delivered can be, for example, from 0 to 200 cycles per second, including about 0 cycles per second, 10 cycles per second, 20 cycles per second, 30 cycles per second, 40 cycles per second, 50 cycles per second, about 60 cycles per second, about 70 cycles per second, about 80 cycles per second, about 90 cycles per second, about 100 cycles per second, about 110 cycles per second, about 120 cycles per second, about 130 cycles per second, about 140 cycles per second, about 150 cycles per second, about 160 cycles per second, about 170 cycles per second, about 180 cycles per second, about 190 cycles per second, and about 200 cycles per second.

### Decellularization

Once formed, the tissue-engineered tubular construct are decellularized to form the tissue-engineered scaffold. Methods for decellularizing structures are known in the art. The decellularization process can comprise a series of sequential extractions. One key feature of this extraction process is that harsh extraction that may disturb or destroy the complex infrastructure of the tissue-engineered tubular construct be avoided. The decellularization can involve a two-step process. The first step can involve removal of cellular debris and solubilization of the cell membrane. This can be followed by solubilization of the nuclear cytoplasmic components and the nuclear components.

The tissue-engineered tubular construct can be decellularized by removing the cell membrane and cellular debris using gentle mechanical disruption methods. The gentle mechanical disruption methods can be sufficient to disrupt the cellular membrane. However, the process of decellularization should avoid damage or disturbance of the biostructure's complex infrastructure. Gentle mechanical disruption methods include scraping the surface of the construct, agitating the construct, or stirring the construct in a suitable volume of fluid, e.g., distilled water. In some cases, the gentle mechanical disruption method can include magnetically stirring (e.g., using a magnetic stir bar and a magnetic plate) the construct in a suitable volume of distilled water until the cell membrane is disrupted and the cellular debris has been removed from the construct.

After the cell membrane has been removed, the nuclear and cytoplasmic components of the construct are removed. This can be performed by solubilizing the cellular and nuclear components without disrupting the infrastructure. To solubilize the nuclear components, non-ionic detergents or surfactants may be used. Examples of non-ionic detergents or surfactants include, but are not limited to, the Triton series, available from Rohm and Haas of Philadelphia, Pa., which includes Triton X-100, Triton N-101, Triton X-114, Triton X-405, Triton X-705, and Triton DF-16, available commercially from many vendors; the Tween series, such as monolaurate (Tween 20), monopalmitate (Tween 40), monooleate (Tween 80), and polyoxethylene-23-lauryl ether (Brij. 35), polyoxyethylene ether W-1 (Polyox), and the like, sodium cholate, deoxycholates, CHAPS, saponin, n-Decyl β-D-glucopuranoside, n-heptyl β-D glucopyranoside, n-Octyl-α-D-glucopyranoside and Nonidet P-40.

One skilled in the art will appreciate that a description of compounds belonging to the foregoing classifications, and vendors may be commercially obtained and may be found in "Chemical Classification, Emulsifiers and Detergents", McCutcheon's, Emulsifiers and Detergents, 1986, North American and International Editions, McCutcheon Division, MC Publishing Co., Glen Rock, N.J., U.S.A. and Judith Neugebauer, A Guide to the Properties and Uses of Detergents in Biology and Biochemistry, Calbiochem, Hoechst Celanese Corp., 1987.

The concentration of the non-ionic detergent may be altered. For example, for delicate constructs, the concentration of detergent can be decreased. Example concentrations ranges of non-ionic detergent can be from about 0.001 to about 2.0% (w/v). If desired, cytoskeletal components comprising dense cytoplasmic filament networks, intercellular complexes, and apical microcellular structures, may be solubilized using an alkaline solution, such as aqueous ammonium hydroxide.

After the decellularization steps described above, the fibers of the scaffold will have retained at least part of the cellularly-deposited ECM produced by the ECM-producing cells. The decellularized, lyophilized scaffold may be stored at a suitable temperature until required for use. Prior to use, the decellularized structure can be equilibrated in suitable isotonic buffer or cell culture medium. Suitable buffers include, but are not limited to, phosphate buffered saline (PBS), saline, MOPS, HEPES, Hank's Balanced Salt Solution, and the like. Suitable cell culture medium includes, but is not limited to, RPMI 1640, Fisher's, Iscove's, McCoy's, Dulbecco's medium, and the like.

In some embodiments, the tissue-engineered scaffold can comprise at least some residual biodegradable synthetic polymer. For example, the tissue-engineered scaffold can comprise at least 0.5% by weight (e.g., from 0.5% to 50% by weight, or from 5% to 50% by weight) residual biodegradable synthetic polymer, based on the total weight of the scaffold. In some of these embodiments, the residual biodegradable synthetic polymer can comprise a polyester, such as polylactic acid (PLA), polyglycolic acid (PGA), poly lactic-co-glycolide (PLGA), polycaprolactone (PCL), polydioxanone (PDS), a polyhydroxyalkanoate (PHA), polyurethane (PU), copolymers thereof, and blends thereof. In certain embodiments, the residual biodegradable synthetic polymer comprises polycaprolactone (PCL).

### Graft Formation

Endothelial cells from the subject are then cultured on the decellularized tissue-engineered scaffold (e.g., in a bioreactor as described above) to form a graft (e.g., autologous vascular grafts, biliary conduits, ureter conduits, etc.) for implantation into a subject.

The endothelial cells can be cultured by seeding the decellularized tissue-engineered scaffold (e.g., the internal surface of the decellularized tissue-engineered scaffold) with endothelial cells, endothelial cell progenitors, or stem cells. In some embodiments, the endothelial cells can be cultured by seeding the decellularized tissue-engineered scaffold with endothelial cells isolated from the subject. For example, the endothelial cells can comprise cells obtained from subcutaneous fat, cells obtained from veins, cells cultured from peripheral blood circulating cells, and combinations thereof. Endothelial cells from relatives or other donors of the same species (i.e., allogenic cells) can also be used with appropriate immunosuppression. Optionally, the tissue-engineered scaffold can be treated with an agent to facilitate adhesion of the endothelial cells, such as fibronectin.

The graft can be conditioned during the culturing step within the bioreactor described above by moving a fluid through the lumen of the graft as a pulsed flow. The pulsed flow can comprise flow at a variable rate (e.g., a rate that increases over time continuously or in a stepwise fashion) to induce a wall shear stress. The pulsed flow can comprise flow at a variable rate (e.g., a rate that increases over time continuously or in a stepwise fashion) to induce a wall shear stress of from 1 dyne/cm² to 30 dyne/cm².

By way of non-limiting illustration, examples of certain embodiments of the present disclosure are given below.

### EXAMPLES

### Example 1: Preparation of Graft Vessel

### Fabrication of Electrospun Scaffold

Tubular electrospun scaffolds will be prepared using a custom designed electrospinning apparatus. The apparatus is schematically illustrated in Figure 1. The scaffold was prepared using a polymer blend that included a synthetic polymer (polyglycolic acid, PGA; Teleflex) and a natural polymer (gelatin; Carbomere). The polymer blend was a 75:25 blend of 12 wt% PGA and gelatin by volume dissolved in 1,1,1,3,3,3-hexafluoro-2-propanol (HFIP, Oakwood Chemical). A porogen solution was prepared that included a water-soluble polymer (polyethelene glycol, PEG; Carbomere) dissolved in chloroform at a concentration of 120% weight per volume. The porogen solution was used to form PEG nanospheres, or particles, within the fibers of the tubular electrospun scaffold which can later be removed by exposing the tubular electrospun scaffold to water. By including the porogen, the porosity of the tubular electrospun scaffold can be dramatically increased, allowing the scaffold to function effectively as a scaffold in subsequent culturing steps.

The electrospinning system included a syringe pump, a high-voltage supply, and a rotating collection mandrel. The polymer blend and porogen solutions were each passed through a positive voltage (14-16 kV) field provided by the power supply. The PGA/gelatin blend solution was delivered through a blunt tip syringe needle at a constant flow rate of 4 mL/h using a syringe pump. The PEG porogen solution was similarly delivered through a blunt tip syringe needle onto the opposing side of the rotating mandrel. The distances between the syringe tips and the mandrel were approximately 10-15 cm. The stainless steel mandrel had a diameter of from 3.0-5.2 mm.

The relative flow rates of the polymer blend solution and the porogen solution could be varied during the course of electrospinning the scaffold to vary the density of porogen particles within the nanofiber matrix. In these examples, the PGA/gelatin polymer blend solution was discharged by itself at a flow rate of 4 mL/h for an initial period of time. Then, the PGA/gelatin polymer blend solution discharge was continued at a constant flow rate of 4 mL/h while the PEG porogen solution was discharged at a variable rate (increasing in intervals from 1 to 4 ml/hr). Once formed, the electrospun matrix formed on the mandrel was soaked in graded solutions of ethanol to water to remove the PEG particles, thereby affording the tubular electrospun scaffold.

### Characterization of the Electrospun Scaffold

The electrospun scaffold was characterized by electron microscopy, thermogravimetric analysis, and Fourier transform infrared spectroscopy (FTIR). In addition, an Instron device was used to perform uniaxial tensile tests on the electrospun tubular scaffold. To simulate radial force, a ringlet of the electrospun tubular scaffold spanned two hooks and the force was recorded until the scaffold failed. The ultimate tensile strength of the scaffold was the point of failure, and was around 1400 KPa.

### Fabrication of the Tissue-Engineered Tubular Construct in a Bioreactor

Human dermal fibroblasts (Lonza) were cultured at passage 3-4 on a T75 flask with advanced DMEM with 10% FBS (Gibco), ascorbic acid (sigma), glutamax (gibco), and penicillin (sigma). The tubular electrospun scaffold was placed in a bioreactor system with or without an internal supporting silicone tube. For the case of the silicone tube: a silicone tube (Saint Gobain) with appropriate inner diameter (depending on the electrospun scaffold) was used as an inner support and was placed through the electrospun scaffold. The electrospun scaffold was sutured to a dacron cuff. This was sterilized in 100% ethanol for 1 hour. The bioreactor was autoclaved for sterilization. The electrospun scaffold was threaded over the side-arms of the bioreactor and the silicone tube was threaded through the side-arms of the bioreactors and attached to connectors. The dacron cuff was tied down to keep the electrospun scaffold over the side-arm. The bioreactor system was soaked in 100% ethanol for additional sterilization, and allowed to dry in a hood overnight. For the case without the silicone tube: the electrospun scaffold was sterilized in 100% ethanol for 1 hour. The bioreactor was autoclaved for sterilization. The electrospun scaffold was threaded over the side-arms of the bioreactor and sutured in place over the side arm. The bioreactor system was soaked in 100% ethanol for additional sterilization, and allowed to dry in a hood overnight.

For the case with the silicone tubing: the bioreactor system was connected to autoclaved tubing to create a flow circuit with a reservoir of PBS with pen/strep to pass through the inner silicone tubing. Next, human dermal fibroblasts at passage 3-4 were used to create a suspension of media containing ~ 5 × 10⁶ (for a length of 6 cm scaffold). The bioreactor culture media was advanced DMEM (Lonza) supplemented with 10% bovine serum (Gibco), Glutamax (Lonza), vitamin C (Sigma), and penicillin (Sigma). The bioreactor culture conditions were static for 2 weeks, and then continued under pulsatile stimulation (at 80- 120 revolutions per minute) or using compressed gas (PSI 3-15, at an interval of 60-100 cycles per minute) for another 8-10 weeks. The media was changed every 5-7 days. For the case without the silicone tube: the bioreactor system was connected to autoclaved tubing to create a flow circuit with a reservoir of bioreactor media to pass through the inside of the electrospun scaffold. Next, human dermal fibroblasts at passage 3-4 were used to create a suspension of media containing ~ 5 × 10⁶ (for a length of 6 cm scaffold). The bioreactor culture conditions were started at a low flow rate (1-5 ml/minute) through the electrospun scaffold. As the electrospun scaffold became more cellular, the flow rate was increased to range of 15-50 ml/minute to provide pulsatile stimulation for another 8-10 weeks.

### Decellularization to Form a Tissue-Engineered Scaffold

The tissue-engineered tubular construct was first treated in a hypertonic solution with 1M NaCl (Sigma) and 25 mM EDTA in PBS (Boston BioProducts) for 1 hour at room temperature. Next, the construct was treated with a solution containing Triton X-100 (Boston BioProducts) 0.5% volume per volume and 0.125% Sodium Deoxychoate (Sigma) weight per volume for 1-2 hours at room temperature. The constructs were then rinsed in a DNAse solution. Finally, the constructs were washed extensively in PBS. All solutions were autoclaved, and all steps were performed under sterile conditions.

### Formation of the Graft

Endothelial cells were obtained to seed the decellularized tissue-engineered scaffold. In order to create an autologous graft, endothelial cells could be obtained from a subject (e.g., from peripheral blood circulating cells that have been cultured, endothelial cells obtained from subcutaneous fat, or from veins) and then cultured on the scaffold. The decellularized tissue-engineered scaffold was pre-coated with fibronectin (10 ug/mL) (BD Bioscience) for 4 hours. A solution of endothelial cells was then seeded into the scaffold lumen at a density of 4-6 × 10⁶ cells / mL, and the scaffold was rotated at 0.5 rpm for 4 hours. Following rotation, the scaffold was incubated statically for 6-12 hours. Following static incubation, the graft was conditioned. The shear conditioning protocol included an initial low shear (1 dyne/cm²) regime with stepwise increase of shear over 24-36 hours to arterial shear rate of 15-25 dynes /cm². The shear stress was calculated by the Poiseuille equation: = (4µQ) / (R³). The flow system included the graft in a flow chamber with a peristaltic pump and a reservoir to act as a dampener.

The methods of the appended claims are not limited in scope by the specific compositions, systems, and methods described herein, which are intended as illustrations of a few aspects of the claims.

The term "comprising" and variations thereof as used herein is used synonymously with the term "including" and variations thereof and are open, non-limiting terms. Although the terms "comprising" and "including" have been used herein to describe various embodiments, the terms "consisting essentially of" and "consisting of" can be used in place of "comprising" and "including" to provide for more specific embodiments of the invention and are also disclosed. Other than where noted, all numbers expressing geometries, dimensions, and so forth used in the specification and claims are to be understood at the very least, and not as an attempt to limit the application of the doctrine of equivalents to the scope of the claims, to be construed in light of the number of significant digits and ordinary rounding approaches.

Unless defined otherwise, all technical and scientific terms used herein have the same meanings as commonly understood by one of skill in the art to which the disclosed invention belongs.

## Claims

1. A method of forming a graft for implantation into a subject comprising:
(i) forming an electrospun scaffold comprising a substantially tubular matrix formed from nanofibers that comprise a blend of a biodegradable synthetic polymer and a biopolymer, wherein the tubular matrix comprises an external surface, an internal surface, and a lumen extending therethrough, wherein the nanofibers have a diameter of from 50 nm to 2000 nm, wherein the tubular matrix has a porosity of at least 50%, as determined by mercury porosimetry or apparent density;
(ii) culturing a population of extracellular matrix-producing cells on the electrospun tubular scaffold to form a tissue-engineered tubular construct;
(iii) decellularizing the tissue-engineered tubular construct to form a tissue-engineered scaffold; and
(iv) culturing endothelial cells from the subject on the tissue-engineered scaffold to form a graft for implantation into the subject.

2. The method of claim 1, wherein the nanofibers comprise from 60-80% by weight biodegradable synthetic polymer and from 10-40% by weight biopolymer.

3. The method of claim 2, wherein the biodegradable synthetic polymer comprises polyglycolic acid (PGA) and the biopolymer comprises gelatin.

4. The method of claim 1, wherein forming the electrospun scaffold comprises
(a) electrically charging a first solution comprising the biodegradable synthetic polymer and the biopolymer;
(b) electrically charging a second solution comprising a porogen;
(c) discharging the electrically charged first solution and the electrically charged second solution onto a grounded target under an electrostatic field, such that the movement of the electrically charged first solution under the electrostatic field causes the electrically charged first solution to evaporate and produce the nanofibers that form the tubular matrix on the grounded target, and the movement of the electrically charged second solution under the electrostatic field causes the electrically charged second solution to evaporate and produce particles comprising the porogen amongst the nanofibers on the grounded target; and
(d) removing the particles comprising the porogen from amongst the nanofibers to form the electrospun scaffold.

5. The method of claim 4, wherein discharging the electrically charged first solution is performed at a constant rate within the range of from 1 ml/hr to 8 ml/hr and wherein discharging the electrically charged second solution is performed at a constant or variable increasing rate within the range of from 1 ml/hr to 8 ml/hr.

6. The method of claim 4, wherein removing the particles comprising the porogen from amongst the nanofibers comprises dissolving the porogen particles in a solvent comprising water, ethanol, or a combination thereof.

7. The method of claim 1, wherein the tubular matrix has a wall thickness of from 500 microns to 1500 microns and the lumen has a diameter of from 2.5 mm to 6.0 mm.

8. The method of claim 1, wherein the electrospun scaffold is substantially free of crosslinkers.

9. The method of claim 1, wherein step (ii) is performed in a bioreactor.

10. The method of claim 1, wherein the tissue-engineered tubular construct is conditioned during step (ii) by moving a fluid through the lumen as a pulsed flow.

11. The method of claim 10, wherein a stretchable tubing extends through the lumen and the graft is conditioned during step (ii) by expanding the stretchable tubing using a compressed fluid and a hydraulic system.

12. The method of claim 1, wherein step (iv) is performed in a bioreactor.

13. The method of claim 1, wherein step (iv) comprises seeding endothelial cells from the subject on the interior surface of the tissue-engineered scaffold.

14. The method of claim 1, wherein the graft is conditioned during step (iv) by moving a fluid through the lumen as a pulsed flow that is varied over time to induce a wall shear stress of from 1 dyne/cm² to 30 dyne/cm².

## Patentansprüche

1. Verfahren zum Herstellen eines Transplantats zum Implantieren in ein Individuum, aufweisend:
(i) Herstellen eines elektrogesponnenen Geflechts, aufweisend eine im Wesentlichen röhrenförmige Matrix, die aus Nanofasern gebildet ist, welche eine Mischung aus einem biologisch abbaubaren synthetischen Polymer und einem Biopolymer aufweisen, wobei die röhrenförmige Matrix eine äußere Oberfläche, eine innere Oberfläche und ein sich dadurch hindurch erstreckendes Lumen aufweist, wobei die Nanofasern einen Durchmesser von 50 nm bis 2000 nm haben, wobei die röhrenförmige Matrix eine Durchlässigkeit von mindestens 50% hat, wie durch Quecksilberporosimetrie oder Schüttgewicht ermittelt;
(ii) Kultivieren einer Population von extrazellulären matrixproduzierenden Zellen auf dem elektrogesponnenen röhrenförmigen Geflecht, um ein durch Gewebezüchtung gebildetes röhrenförmiges Konstrukt herzustellen;
(iii) Dezellularisieren des durch Gewebezüchtung gebildeten röhrenförmigen Konstrukts zur Bildung eines durch Gewebezüchtung gebildeten Geflechts; und
(iv) Kultivieren von Endothelzellen des Individuums auf dem durch Gewebezüchtung gebildeten Geflecht zum Herstellen eines Transplantats zum Implantieren in das Individuum.

2. Verfahren nach Anspruch 1, wobei die Nanofasern 60 bis 80 Gewichtsprozent biologisch abbaubares synthetisches Polymer und 10 bis 40 Gewichtsprozent Biopolymer aufweisen.

3. Verfahren nach Anspruch 2, wobei das biologisch abbaubare synthetische Polymer Polyglykolsäure (PGA) und das Biopolymer Gelatine aufweist.

4. Verfahren nach Anspruch 1, wobei das Herstellen des elektrogesponnenen Geflechts aufweist:
(a) elektrisches Aufladen einer ersten Lösung, die das biologisch abbaubare synthetische Polymer und das Biopolymer aufweist;
(b) elektrisches Aufladen einer zweiten Lösung, die ein Porogen aufweist;
(c) Entladen der elektrisch aufgeladenen ersten Lösung und der elektrisch aufgeladenen zweiten Lösung auf ein geerdetes Zielobjekt unter einem elektrostatischen Feld, derart, dass die Bewegung der elektrisch aufgeladenen ersten Lösung unter dem elektrostatischen Feld bewirkt, dass die elektrisch aufgeladene erste Lösung verdampft und diejenigen Nanofasern erzeugt, welche die röhrenförmige Matrix auf dem geerdeten Zielobjekt bilden, und die Bewegung der elektrisch aufgeladenen zweiten Lösung unter dem elektrostatischen Feld bewirkt, dass die elektrisch aufgeladene zweite Lösung verdampft und Partikel erzeugt, welche unter den Nanofasern auf dem geerdeten Zielobjekt das Porogen enthalten; und
(d) Entfernen der das Porogen enthaltenden Partikel unter den Nanofasern, um das elektrogesponnene Geflecht herzustellen.

5. Verfahren nach Anspruch 4, wobei das Entladen der elektrisch aufgeladenen ersten Lösung bei einer konstanten Rate innerhalb des Bereichs von 1 ml/h bis 8 ml/h durchgeführt wird und wobei das Entladen der elektrisch aufgeladenen zweiten Lösung bei einer konstanten oder variabel zunehmenden Rate innerhalb des Bereichs von 1 ml/h bis 8 ml/h durchgeführt wird.

6. Verfahren nach Anspruch 4, wobei das Entfernen der das Porogen enthaltenden Partikel unter den Nanofasern das Auflösen der Porogen-Partikel in einem Wasser, Ethanol oder eine Kombination daraus enthaltenden Lösungsmittel umfasst.

7. Verfahren nach Anspruch 1, wobei die röhrenförmige Matrix eine Wanddicke von 500 µm bis 1500 µm und das Lumen einen Durchmesser von 2,5 mm bis 6,0 mm hat.

8. Verfahren nach Anspruch 1, wobei das elektrogesponnene Geflecht im Wesentlichen frei von Vernetzungsmitteln ist.

9. Verfahren nach Anspruch 1, wobei Schritt (ii) in einem Bioreaktor durchgeführt wird.

10. Verfahren nach Anspruch 1, wobei das durch Gewebezüchtung gebildete röhrenförmige Konstrukt in Schritt (ii) durch Bewegen einer Flüssigkeit als gepulster Fluss durch das Lumen aufbereitet wird.

11. Verfahren nach Anspruch 10, wobei sich ein dehnbarer Schlauch durch das Lumen erstreckt und das Transplantat in Schritt (ii) durch Erweitern des dehnbaren Schlauchs unter Verwendung einer komprimierten Flüssigkeit und eines hydraulischen Systems aufbereitet wird.

12. Verfahren nach Anspruch 1, wobei Schritt (iv) in einem Bioreaktor durchgeführt wird.

13. Verfahren nach Anspruch 1, wobei Schritt (iv) die Aussaat von Endothelzellen des Individuums auf der inneren Oberfläche des durch Gewebezüchtung gebildeten Geflechts umfasst.

14. Verfahren nach Anspruch 1, wobei das Transplantat in Schritt (iv) aufbereitet wird, indem eine Flüssigkeit als gepulster Fluss durch das Lumen bewegt wird, wobei der gepulste Fluss über die Zeit variiert wird, um eine Wandscherspannung von 1 Dyne/cm² bis 30 Dyne/cm² zu erzeugen.

## Revendications

1. Procédé de formation d'un greffon pour implantation chez un sujet, comprenant :
(i) la formation d'un support électrofilé comprenant une matrice sensiblement tubulaire formée à partir de nanofibres qui comprennent un mélange d'un polymère synthétique biodégradable et d'un biopolymère, où la matrice tubulaire comprend une surface externe, une surface interne, et une lumière s'étendant à travers celle-ci, où les nanofibres ont un diamètre compris entre 50 nm et 2 000 nm, où la matrice tubulaire a une porosité d'au moins 50 %, telle que déterminée par porosimétrie au mercure ou densité apparente ;
(ii) la culture d'une population de cellules extracellulaires productrices de matrice sur le support tubulaire électrofilé pour former une construction tubulaire obtenue par ingénierie tissulaire ;
(iii) la décellularisation de la construction tubulaire obtenue par ingénierie tissulaire pour former un support obtenu par ingénierie tissulaire ; et
(iv) la culture de cellules endothéliales provenant du sujet sur le support obtenu par ingénierie tissulaire pour former un greffon pour implantation chez le sujet.

2. Procédé de la revendication 1, dans lequel les nanofibres comprennent 60 à 80 % en poids de polymère synthétique biodégradable et 10 à 40 % en poids de biopolymère.

3. Procédé de la revendication 2, dans lequel le polymère synthétique biodégradable comprend de l'acide polyglycolique (PGA), et le biopolymère comprend de la gélatine.

4. Procédé de la revendication 1, dans lequel la formation du support électrofilé comprend
(a) le chargement électrique d'une première solution comprenant le polymère synthétique biodégradable et le biopolymère ;
(b) le chargement électrique d'une seconde solution comprenant un porogène ;
(c) la décharge de la première solution chargée électriquement et de la seconde solution chargée électriquement sur une cible mise à la terre sous un champ électrostatique, de telle sorte que le mouvement de la première solution chargée électriquement sous le champ électrostatique provoque l'évaporation de la première solution chargée électriquement et la production de nanofibres qui forment la matrice tubulaire sur la cible mise à la terre, et le mouvement de la seconde solution chargée électriquement sous le champ électrostatique provoque l'évaporation de la seconde solution chargée électriquement et la production de particules comprenant le porogène parmi les nanofibres sur la cible mise à la terre ; et
(d) l'élimination des particules comprenant le porogène au sein des nanofibres pour former le support électrofilé.

5. Procédé selon la revendication 4, dans lequel la décharge de la première solution chargée électriquement est effectuée selon un débit constant dans la plage allant de 1 ml/h à 8 ml/h, et dans lequel la décharge de la seconde solution électriquement chargée est effectuée selon un débit constant ou variable et croissant dans la plage allant de 1 ml/h à 8 ml/h.

6. Procédé de la revendication 4, dans lequel l'élimination des particules comprenant le porogène des nanofibres comprend la dissolution des particules porogènes dans un solvant comprenant de l'eau, de l'éthanol, ou une combinaison de ceux-ci.

7. Procédé de la revendication 1, dans lequel la matrice tubulaire a une épaisseur de paroi allant de 500 microns à 1 500 microns, et la lumière a un diamètre allant de 2,5 mm à 6,0 mm.

8. Procédé de la revendication 1, dans lequel le support électrofilé est sensiblement exempte d'agents de réticulation.

9. Procédé de la revendication 1, dans lequel l'étape (ii) est réalisée dans un bioréacteur.

10. Procédé de la revendication 1, dans lequel la construction tubulaire obtenue par ingénierie tissulaire est conditionnée pendant l'étape (ii) en déplaçant un fluide à travers la lumière sous la forme d'un flux pulsé.

11. Procédé de la revendication 10, dans lequel un tube étirable s'étend à travers la lumière, et le greffon est conditionné pendant l'étape (ii) en agrandissant le tube étirable à l'aide d'un fluide comprimé et d'un système hydraulique.

12. Procédé de la revendication 1, dans lequel l'étape (iv) est réalisée dans un bioréacteur.

13. Procédé de la revendication 1, dans lequel l'étape (iv) comprend l'ensemencement de cellules endothéliales provenant du sujet sur la surface intérieure du support obtenu par ingénierie tissulaire.

14. Procédé de la revendication 1, dans lequel le greffon est conditionné au cours de l'étape (iv) en déplaçant un fluide à travers la lumière sous la forme d'un flux pulsé qui varie avec le temps pour induire une contrainte de cisaillement de paroi allant de 1 dyne/cm² à 30 dynes/cm².
